# EUROPEAN PATENT APPLICATION

(11) **EP 4 467 069 A1**
(43) Date of publication of application: **27.11.2024**
(21) Application number: 24177317.5
(22) Date of filing: 22.05.2024
(51) Int. Cl.: A61B 5/021, A61B 5/022, A61B 5/024, A61B 5/00, G16H 50/00

(54) **DEEP LEARNING-BASED CONTINUOUS ARTERIAL BLOOD PRESSURE MONITORING SYSTEM AND METHOD BASED ON PHOTOPLETHYSMOGRAPHY AND NON-INVASIVE BLOOD PRESSURE MEASUREMENTS**

(30) Priority: 22.05.2023 KR 20230065750
(71) Applicant: Seoul National University Hospital, Seoul 03080 (KR)
(72) Inventor: LEE, Hyung-Chul, 05504 Seoul (KR); LEE, Hyeonhoon, 02851 Seoul (KR)
(74) Representative: Mewburn Ellis LLP

(57) **Abstract**

A continuous arterial blood pressure monitoring system includes a data receiving unit that receives a photoplethysmography measurement value and a non-invasive blood pressure measurement value, a photoplethysmography analysis unit that acquires a primary arterial blood pressure waveform from the photoplethysmography measurement value using a first learning model, a derived variable extraction unit that extracts a derived variable using the non-invasive blood pressure measurement value, a non-invasive blood pressure analysis unit that inputs the derived variable and the non-invasive blood pressure measurement value to a second learning model and extracts a second feature value, a blood pressure waveform prediction unit that inputs the primary arterial blood pressure waveform and the second feature values to a third learning model, and predicts the continuous arterial blood pressure waveform, and a blood pressure state prediction unit that predicts the blood pressure state of a patient using the continuous arterial blood pressure waveform.

## Description

### CROSS-REFERENCE TO RELATED APPLICATION

This application is based on and claims priority under 35 U.S.C. §119 to Korean Patent Application No. 10-2023-0065750, filed on MAY 22, 2023, in the Korean Intellectual Property Office, the disclosure of which is incorporated by reference herein in its entirety.

### BACKGROUND

The present disclosure relates to a deep learning-based continuous arterial blood pressure monitoring system and a method thereof using a photoplethysmography and a non-invasive blood pressure measurement value and more specifically, relates to a continuous arterial blood pressure monitoring system and a method thereof for estimating continuous arterial blood pressure waveforms from a photoplethysmography and a non-invasive biosignal pattern including a non-invasive blood pressure, and classifying a blood pressure state by using the estimated arterial blood pressure waveforms.

Blood pressure may be measured by using an upper arm sphygmomanometer or a sensor inserted into smart clothing, a smart watch, or the like.

A blood pressure measurement method using the upper arm sphygmomanometer is a non-invasive blood pressure measurement method that measures the blood pressure by wrapping a cuff around the arm and pressurizing the cuff. To explain this again, the upper arm sphygmomanometer measures systolic and diastolic blood pressures by applying a pressure large enough to block the artery and then using sound of the vortex that occurs when blood flows while slowly reducing the pressure. Although the method is less expensive and less risky than an invasive measurement method, it has the disadvantage of not being able to obtain information about continuous changes in blood pressure unless repeated measurements are made.

Therefore, conventionally, the continuous blood pressure was measured by using the invasive method of inserting a device into the human body and measuring the blood pressure directly in the artery. The invasive blood pressure measurement method is useful when a hemodynamic state is unstable or arterial blood analysis is continuously required, so the blood pressure is mainly measured for the diagnosis and management of cardiovascular disease and the invasive blood pressure measurement method is used when continuous monitoring is required for a critical patient or the like. However, the invasive insertion into the radial or femoral artery carries risks of infection, bleeding, pain, and blood vessel damage.

In addition, the method of measuring the blood pressure by using a sensor is a method of measuring the blood pressure by using the photoplethysmography.

Here, the photoplethysmography is a technology for measuring changes in the blood flow in blood vessels near the skin, and changes in blood flow are detected by measuring how much light is absorbed and reflected by using an LED sensor. In addition, through the LED sensor, a specific algorithm is applied to measure various biological signals such as a heart rate and a breathing rate. Therefore, the photoplethysmography signal serves as a major variable in estimating the blood pressure waveform.

Meanwhile, there is a problem that an overall blood pressure estimation accuracy is low due to auto-calibration in a signal device such as the photoplethysmography signal, noise in the mechanical device itself, or the like, or it results in inaccurate prediction in a specific blood pressure range.

An example of related art includes Korean Patent Publication No. 10-2023-0025177 (published on February 21, 2023).

### SUMMARY

The present disclosure provides a continuous arterial blood pressure monitoring system and a method thereof for estimating continuous arterial blood pressure waveforms from a photoplethysmography and a non-invasive biosignal pattern including a non-invasive blood pressure, and classifying a blood pressure state by using the estimated arterial blood pressure waveforms.

According to an aspect of the present disclosure, a deep learning-based continuous arterial blood pressure monitoring system using a photoplethysmography and a non-invasive blood pressure measurement value includes a data receiving unit configured to receive a photoplethysmography (PPG) measurement value and a non-invasive blood pressure measurement value of a patient whose blood pressure state is to be analyzed, a photoplethysmography analysis unit configured to acquire a primary arterial blood pressure waveform from the photoplethysmography measurement value using a first learning model, a derived variable extraction unit configured to extract a derived variable using the received non-invasive blood pressure measurement value, a non-invasive blood pressure analysis unit configured to input the extracted derived variable and the non-invasive blood pressure measurement value to a second learning model and extract a second feature value, a blood pressure waveform prediction unit configured to input the primary arterial blood pressure waveform and the second feature values to a third learning model and predict the continuous arterial blood pressure waveform, and a blood pressure state prediction unit configured to predict the blood pressure state of the patient using the predicted continuous arterial blood pressure waveform.

The derived variable extraction unit may generate a derived variable according to the time elapse from a time point of measurement of the most recently measured non-invasive blood pressure measurement value to a time point to be predicted.

The blood pressure waveform prediction unit may input the primary arterial blood pressure waveform and the second feature value to the constructed third learning model and generate a continuous final blood pressure waveform.

The data receiving unit may receive the patient's photoplethysmography measurement value and non-invasive blood pressure measurement value measured through a serial port or a Lan port of a commercial monitoring device.

The continuous arterial blood pressure monitoring system may further include a data transmitting unit configured to convert the predicted continuous arterial blood pressure waveform into a voltage using a D-A converter and transmit the predicted continuous arterial blood pressure waveform to a blood pressure measurement module of a commercial monitoring device, or transmit the continuous arterial blood pressure waveform in digital form to a central monitoring device or an automatic medical record system (electronic medical records).

In addition, according to another aspect of the present disclosure, a monitoring method of an arterial blood pressure using an arterial blood pressure monitoring system, includes receiving a photoplethysmography (PPG) measurement value and a non-invasive blood pressure measurement value of a patient whose blood pressure state is to be analyzed, acquiring a primary arterial blood pressure waveform from the photoplethysmography measurement value using a first learning model, extracting a derived variable using the received non-invasive blood pressure measurement value, inputting the extracted derived variable and the non-invasive blood pressure measurement value to a second learning model, and extracting a second feature value, inputting the primary arterial blood pressure waveform and the second feature values to a third learning model, and predicting the continuous arterial blood pressure waveform, and predicting the blood pressure state of the patient using the predicted continuous arterial blood pressure waveform.

### BRIEF DESCRIPTION OF THE DRAWINGS

Embodiments of the inventive concept will be more clearly understood from the following detailed description taken in conjunction with the accompanying drawings in which:
FIG. 1 is a configuration diagram illustrating a continuous arterial blood pressure monitoring system according to an embodiment of the present disclosure;
FIG. 2 is a flowchart illustrating a monitoring method of a continuous arterial blood pressure by using a continuous arterial blood pressure monitoring system according to an embodiment of the present disclosure;
FIG. 3 is an exemplary diagram for explaining step S240 illustrated in FIG. 2;
FIG. 4 is an exemplary diagram illustrating a state in which a final blood pressure waveform and a classified blood pressure state output in step S260 illustrated in FIG. 2 are transmitted to an external monitoring device;
FIG. 5 is a diagram illustrating performance evaluation indices of a model that does not consider a non-invasive blood pressure (NIBP) and a model that considers the non-invasive blood pressure (NIBP) according to an embodiment of the present disclosure; and
FIG. 6 is an exemplary diagram of blood pressure waveforms predicted by using the models illustrated in FIG. 5.

### DETAILED DESCRIPTION OF THE EMBODIMENTS

Hereinafter, with reference to the attached drawings, embodiments of the present disclosure will be described in detail so that those skilled in the art may easily implement the present disclosure. However, the present disclosure may be implemented in many different forms and is not limited to the embodiments described herein. In order to clearly explain the present disclosure in the drawings, parts that are not related to the description are omitted, and similar parts are given similar reference numerals throughout the specification.

Throughout the specification, when a part is said to "include" a certain element, this means that it may further include other elements rather than excluding other elements, unless specifically stated to the contrary.

Then, with reference to the attached drawings, embodiments of the present disclosure will be described in detail so that those skilled in the art may easily implement the present disclosure.

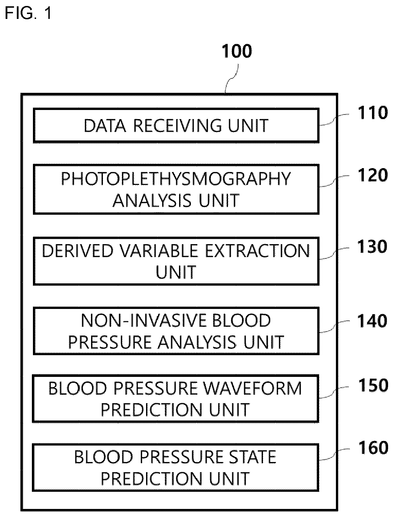

Hereinafter, a continuous arterial blood pressure monitoring system according to an embodiment of the present disclosure will be described in more detail with reference to FIG. 1.

FIG. 1 is a configuration diagram illustrating the continuous arterial blood pressure monitoring system according to an embodiment of the present disclosure.

As illustrated in FIG. 1, a continuous arterial blood pressure monitoring system 100 according to an embodiment of the present disclosure includes a data receiving unit 110, a photoplethysmography analysis unit 120, a derived variable extraction unit 130, and a non-invasive blood pressure analysis unit 140, a blood pressure waveform prediction unit 150, and a blood pressure state prediction unit 160.

First, the data receiving unit 110 receives a photoplethysmography measurement value measured through a photoplethysmography sensor. In addition, the data receiving unit 110 receives a non-invasive blood pressure measurement value measured through a sphygmomanometer.

The photoplethysmography analysis unit 120 normalizes the received photoplethysmography measurement value, inputs the normalized photoplethysmography measurement value to a first learning model, and acquires a primary arterial blood pressure waveform.

The derived variable extraction unit 130 extracts derived variables according to the time elapse from the time point of measurement of the most recently measured non-invasive blood pressure measurement value to the time point to be predicted.

The non-invasive blood pressure analysis unit 140 inputs the extracted derived variable and the corresponding blood pressure measurement value to a second learning model and extracts a second feature value.

The blood pressure waveform prediction unit 150 predicts the continuous arterial blood pressure waveform by using the primary arterial blood pressure waveform acquired through the photoplethysmography analysis unit 120 and the second feature value extracted through the non-invasive blood pressure analysis unit 140. To elaborate, the blood pressure waveform prediction unit 150 inputs the first and second feature values for the photoplethysmography to a third learning model including a cross-attention layer, and outputs the continuous arterial blood pressure waveform.

The blood pressure state prediction unit 160 predicts the blood pressure state by using the output continuous arterial blood pressure waveform.

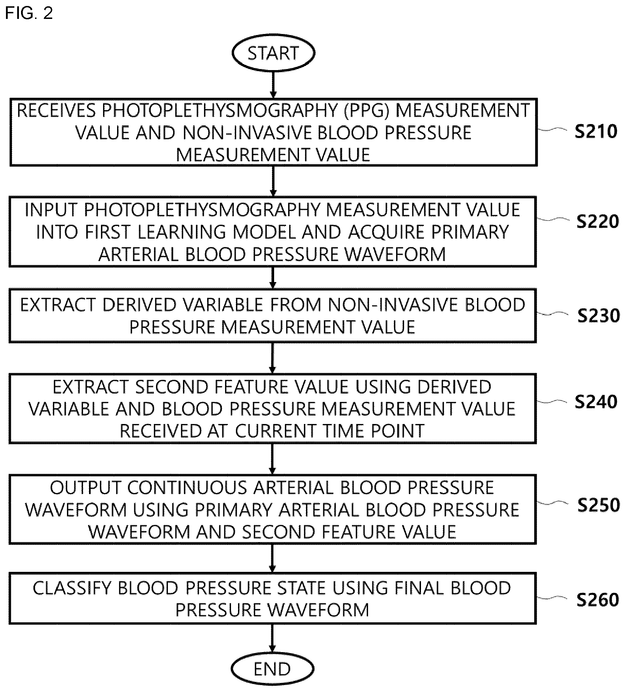

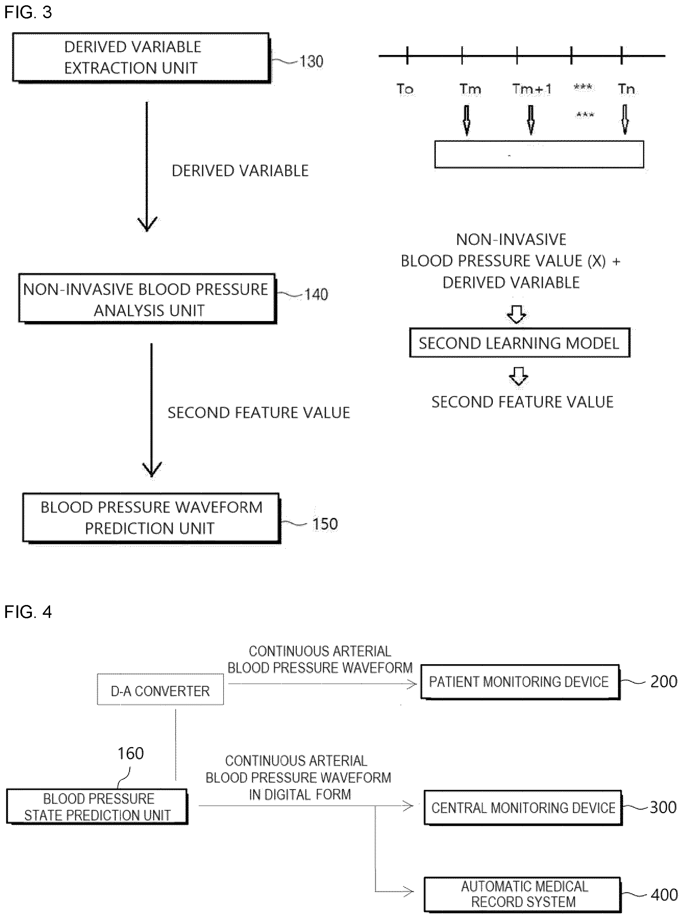

Hereinafter, a monitoring method of a continuous arterial blood pressure by using the continuous arterial blood pressure monitoring system 100 according to an embodiment of the present disclosure will be described in more detail with reference to FIGS. 2 and 3.

FIG. 2 is a flowchart illustrating a monitoring method of a continuous arterial blood pressure by using a continuous arterial blood pressure monitoring system according to an embodiment of the present disclosure.

As illustrated in FIG. 2, the continuous arterial blood pressure monitoring system 100 according to an embodiment of the present disclosure receives a photoplethysmography measurement value and a non-invasive blood pressure measurement value of a patient whose blood pressure state is to be analyzed (S210).

In details, the data receiving unit 110 receives the photoplethysmography measurement values from a smart healthcare device worn by the patient or a patient monitoring device used in the hospital. At this time, the smart healthcare device is equipped with a photoplethysmography sensor, which measures the photoplethysmography by absorbing, reflecting, and scattering light based on the change in blood volume within the blood vessel according to the heartbeat.

In addition, the data receiving unit 110 receives the non-invasive blood pressure measurement value measured by applying a pressure to the blood vessel while a cuff is worn on a part of the patient's body. Here, the non-invasive blood pressure measurement value includes the systolic blood pressure value and the diastolic blood pressure value.

In addition, the data receiving unit 110 may receive the patient's photoplethysmography measurement value and non-invasive blood pressure measurement value measured through a serial port or a Lan port of a commercial monitoring device.

When step S210 is completed, the photoplethysmography analysis unit 120 inputs the received photoplethysmography measurement value to the first learning model and acquires the primary arterial blood pressure waveform (S220).

Here, the first learning model may be any algorithm as long as it is a deep learning-based algorithm such as Resnet algorithm, U-net algorithm, Attention algorithm, and Self-Attention algorithm. That is, the algorithm applied to the first learning model is not limited to a few algorithms, but the first learning model is constructed by using one or more algorithms among various known algorithms that may be applied by a person skilled in the art.

The photoplethysmography sensor measures the photoplethysmography in a state of being attached to any one body part among fingertips, wrists, fingers, ears, head, and ankles. At this time, the photoplethysmography measurement value may change depending on external environmental factors as well as the measuring location. Accordingly, the photoplethysmography analysis unit 120 normalizes the photoplethysmography measurement value, and then the normalized photoplethysmography measurement value is input to the previously constructed first learning model.

The first learning model consists of multiple convolutional layers to extract features from the encoding region and decoding region, stores the features of the photoplethysmography extracted in multiple encoding steps, and then uses the features of the stored photoplethysmography. Then, a decoding step is performed to output the primary arterial blood pressure waveform.

When step S220 is completed, the derived variable extraction unit 130 extracts the derived variable from the non-invasive blood pressure measurement value (S230).

The derived variable extraction unit 130 generates derived variables according to the time elapse from the time point of measurement of the most recently measured non-invasive blood pressure measurement value to the time point to be predicted. To explain this again, in order to determine how much influence the non-invasive blood pressure measurement value measured in the past has on the time point to be predicted, the derived variable for a length of the photoplethysmography measurement value is generated based on the current time point to be predicted from the time point when the most recent non-invasive blood pressure measurement was made. For example, it is assumed that the section from Tm to Tn is the section to be predicted, and that there are N time points within the section. Then, the derived variable extraction unit 130 generates the derived variable for how much time has elapsed since the measurement time point To of the most recently measured non-invasive blood pressure measurement value for each N time points included in the section from Tm to Tn. Here N=(n-m+1). In other words, each derived variable is generated at each time point that exists within the section.

Next, the non-invasive blood pressure analysis unit 140 extracts the second feature value by using the generated derived variable and the blood pressure measurement value received at the current time point (S240).

FIG. 3 is an exemplary diagram for explaining step S240 illustrated in FIG. 2.

As illustrated in FIG. 3, the non-invasive blood pressure analysis unit 140 inputs the non-invasive blood pressure measurement value X corresponding to the generated derived variable to the second learning model.

For example, it is assumed that the most recently measured non-invasive blood pressure measurement time point is To, the prediction start time point is Tm, and the current time point to be predicted is Tn. In order to check how much influence the non-invasive blood pressure measurement value measured in the past has on the time point to be predicted, in an embodiment of the present disclosure, the derived variable for a length (n-m+1) of the photoplethysmography measurement value is generated based on the time point Tn to be predicted from the most recently measured non-invasive blood pressure measurement time point To. Next, the non-invasive blood pressure analysis unit 140 inputs the same number of non-invasive blood pressure values X as the number of generated derived variables to the second learning model. Then, the second learning model outputs the second feature value corresponding to the input value. Here, like the first learning model, the second learning model may be constructed by using one or more of several known algorithms such as the LSTM algorithm.

In an embodiment of the present disclosure, it is described that the step S220 of analyzing the photoplethysmography measurement value is preceded and then the steps S230 and S240 of analyzing the non-invasive blood pressure measurement value are processed, but the steps may be carried out simultaneously or the step of analyzing the non-invasive blood pressure measurement value may be preceded.

When steps S220 to S240 are completed, the blood pressure waveform prediction unit 150 outputs the continuous arterial blood pressure waveform by using the primary arterial blood pressure waveform and the second feature value (S250).

First, the blood pressure waveform prediction unit 150 builds a third learning model including the Cross-Attention algorithm and the forward neural network algorithm. Next, the blood pressure waveform prediction unit 150 inputs the primary arterial blood pressure waveform and the second feature value to the constructed third learning model and generates a continuous final blood pressure waveform. The final blood pressure waveform generated here represents the continuous arterial blood pressure waveform.

When step S250 is completed, the blood pressure state prediction unit 160 classifies the blood pressure state by using the generated final blood pressure waveform (S260).

The blood pressure state prediction unit 160 determines the systolic blood pressure (SBP) and the diastolic blood pressure (DBP) from the generated final blood pressure waveform. Next, the blood pressure state prediction unit 160 uses the determined systolic blood pressure and diastolic blood pressure to classify the blood pressure state of the patient into any one of low blood pressure, normal, prehypertension, and high blood pressure.

FIG. 4 is an exemplary diagram illustrating a state in which the final blood pressure waveform and the classified blood pressure state output in step S260 illustrated in FIG. 2 are transmitted to an external monitoring device.

As illustrated in FIG. 4, the blood pressure state prediction unit 160 may transmit the final blood pressure waveform and the blood pressure state to a patient monitoring device 200, a central monitoring device 300, or an automatic medical record system (electronic medical records) 400 through a data transmitting unit (not illustrated).

In order to elaborate, the data transmitting unit may convert the predicted continuous arterial blood pressure waveform into a voltage by using a D-A converter and transmit it to a blood pressure measurement module of the patient monitoring device 200 which is a commercial monitoring device.

In addition, the data transmitting unit may transmit a continuous arterial blood pressure waveform in digital form to the central monitoring device 300 or the automatic medical record system 400.

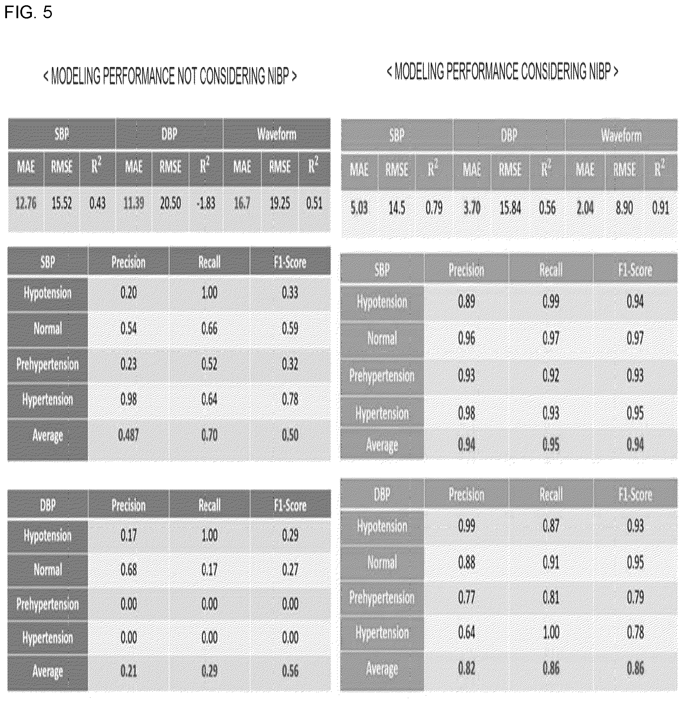

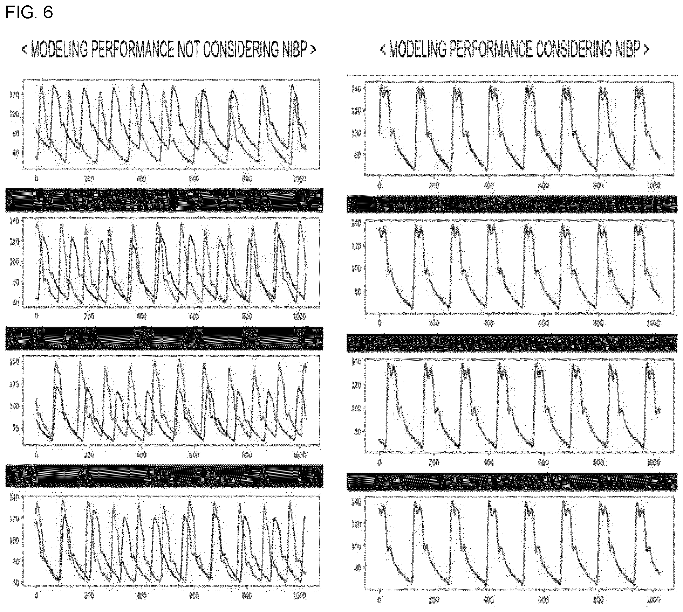

Hereinafter, performance evaluation results of the continuous arterial blood pressure monitoring system according to an embodiment of the present disclosure will be described in more detail with reference to FIGS. 5 and 6.

The continuous arterial blood pressure monitoring system according to the embodiment of the present disclosure is characterized by predicting the continuous arterial blood pressure by considering both the photoplethysmography (PPG) and non-invasive blood pressure (NIBP).

FIG. 5 is a diagram illustrating performance evaluation indices of a model that does not consider a non-invasive blood pressure and a model that considers the non-invasive blood pressure according to an embodiment of the present disclosure, and FIG. 6 is an exemplary diagram of blood pressure waveforms predicted by using the models illustrated in FIG. 5.

As illustrated in FIG. 5, it may be seen that in the model that considers the non-invasive blood pressure according to the embodiment of the present disclosure (right and expressed in orange), mean absolute error (MAE), precision, recall, and F1-score are all further improved compared to those in the model that does not consider the non-invasive blood pressure (left and expressed in gray).

A blue waveform illustrated in FIG. 6 is a waveform representing a measured value (A-line) of an actually measured invasive continuous arterial blood pressure waveform, and a red waveform is an A-line waveform representing a predicted result. As a result, it may be seen that the waveform output from the model considering the non-invasive blood pressure according to the embodiment of the present disclosure is almost identical to the actually measured waveform.

As described above, the continuous arterial blood pressure monitoring system according to the present disclosure may utilize the biosignal measured through the non-invasive sensor device, and estimate the continuous arterial blood pressure waveforms by using an artificial intelligence algorithm, and thereby it is possible to early predict the cardiovascular disease.

The present disclosure is described with reference to the embodiments illustrated in the drawings, but these are merely illustrative, and those skilled in the art will understand that various modifications and equivalent other embodiments may be derived therefrom. Therefore, the true scope of technical protection of the present disclosure should be determined by the technical idea of the attached patent claims.

## Claims

1. A deep learning-based continuous arterial blood pressure monitoring system using a photoplethysmography and a non-invasive blood pressure measurement value, the continuous arterial blood pressure monitoring system comprising:
a data receiving unit configured to receive a photoplethysmography (PPG) measurement value and a non-invasive blood pressure measurement value of a patient whose blood pressure state is to be analyzed;
a photoplethysmography analysis unit configured to acquire a primary arterial blood pressure waveform from the photoplethysmography measurement value using a first learning model;
a derived variable extraction unit configured to extract a derived variable using the received non-invasive blood pressure measurement value;
a non-invasive blood pressure analysis unit configured to input the extracted derived variable and the non-invasive blood pressure measurement value to a second learning model and extracts a second feature value;
a blood pressure waveform prediction unit configured to input the primary arterial blood pressure waveform and the second feature values to a third learning model, and predict the continuous arterial blood pressure waveform; and
a blood pressure state prediction unit configured to predict the blood pressure state of the patient using the predicted continuous arterial blood pressure waveform.

2. The continuous arterial blood pressure monitoring system according to claim 1, wherein
the derived variable extraction unit generates a derived variable according to the time elapse from a time point of measurement of the most recently measured non-invasive blood pressure measurement value to a time point to be predicted.

3. The continuous arterial blood pressure monitoring system according to claim 1, wherein
the blood pressure waveform prediction unit inputs the primary arterial blood pressure waveform and the second feature value to the constructed third learning model and generates a continuous final blood pressure waveform.

4. The continuous arterial blood pressure monitoring system according to claim 1, wherein
the data receiving unit receives the patient's photoplethysmography measurement value and non-invasive blood pressure measurement value measured through a serial port or a Lan port of a commercial monitoring device.

5. The continuous arterial blood pressure monitoring system according to claim 1, further comprising:
a data transmitting unit configured to convert the predicted continuous arterial blood pressure waveform into a voltage using a D-A converter and transmits the predicted continuous arterial blood pressure waveform to a blood pressure measurement module of a commercial monitoring device, or transmits the continuous arterial blood pressure waveform in digital form to a central monitoring device or an automatic medical record system (electronic medical records).

6. A monitoring method of an arterial blood pressure using an arterial blood pressure monitoring system, the monitoring method comprising:
receiving a photoplethysmography (PPG) measurement value and a non-invasive blood pressure measurement value of a patient whose blood pressure state is to be analyzed;
acquiring a primary arterial blood pressure waveform from the photoplethysmography measurement value using a first learning model;
extracting a derived variable using the received non-invasive blood pressure measurement value;
inputting the extracted derived variable and the non-invasive blood pressure measurement value to a second learning model, and extracting a second feature value;
inputting the primary arterial blood pressure waveform and the second feature values to a third learning model, and predicting the continuous arterial blood pressure waveform; and
predicting the blood pressure state of the patient using the predicted continuous arterial blood pressure waveform.
